# EUROPEAN PATENT SPECIFICATION

(11) **EP 4 429 691 B1**
(45) Date of publication and mention of the grant of the patent: **25.02.2026**
(21) Application number: 21966769.8
(22) Date of filing: 09.12.2021
(51) Int. Cl.: A61K 38/13, A61P 17/02

(54) **WS635 FOR PROMOTING WOUND HEALING**
WS635 ZUR FÖRDERUNG DER WUNDHEILUNG
WS635 POUR FAVORISER LA CICATRISATION DES PLAIES

(43) Date of publication of application: 18.09.2024
(73) Proprietor: Waterstone Pharmaceuticals (Wuhan) Co., Ltd., Wuhan, Hubei 430075 (CN)
(72) Inventor: ZHANG, Faming, Wuhan, Hubei 430075 (CN); SHEN, Yuan, Wuhan, Hubei 430075 (CN); HU, Minglong, Wuhan, Hubei 430075 (CN); YU, Yao, Wuhan, Hubei 430075 (CN); WANG, Xiaolong, Wuhan, Hubei 430075 (CN); ZHAO, Along, Wuhan, Hubei 430075 (CN)
(74) Representative: Isarpatent
(86) International application number: PCT/CN2021/136859
(87) International publication number: WO 2023/102847

(56) References cited:
- WO-A1-2021/068188
- CN-A- 106 902 347
- CN-A- 108 348 468
- CN-A- 110 678 187
- KONG WUYI ET AL: "Cyclophilin C-associated protein is up-regulated during wound healing", JOURNAL OF CELLULAR PHYSIOLOGY, vol. 210, no. 1, 22 September 2006 (2006-09-22), US, pages 153 - 160, XP093228629, ISSN: 0021-9541, DOI: 10.1002/jcp.20830
- YAO GUIYANG ET AL.: "Nonimmunosuppressive Cyclophilin Inhibitors Derived from the Cyclosporin Scaffolds.", JOURNAL OF INTEGRATION TECHNOLOGY, vol. 4, no. 4, 31 July 2015 (2015-07-31), pages 28 - 44, XP093068847, DOI: 10.12146/j.issn.2095-3135.201504004

## Description

### FIELD OF THE DISCLOSURE

The present invention belongs to the field of medicine. Specifically, it relates to (3S,6S,9S,12R,15S,18S,21S,24S,27R,30S,33S)-27-((2-(dimethylamino)ethyl)thio)-30-ethyl-33-((1R,2R,E)-1-hydroxy-2-methylhex-4-en-1-yl)-24-(2-hydroxy-2-methylpropyl)-6,9,18-triisobutyl-3,21-diisopropyl-1,4,7,10,12,15,19,25,28-nonamethyl-1,4,7,10,13,16,19,22,25,28,31-undecaazacyclotritriacontan-2,5,8,11,14,17,20,23,26,29,32-undecaone (I) (WS635, also known as SCY-635) and pharmaceutically compositions thereof for use in a method of promoting a healing process of wound in a patient.

### BACKGROUND OF THE INVENTION

The skin forms a physical barrier, which is essential to prevent microbial invasion and maintain temperature and fluid balance. Skin wound can temporarily destroy this barrier and thus pose a huge health challenge. Wound healing is a complex process, which is characterized by the dynamic changes of the wound microenvironment recruiting and guiding different types of participating cells. The entire healing process of wound is typically divided into four consecutive overlapping stages: hemostasis, inflammation, proliferation, and remodeling. In the hemostasis phase, bleeding is controlled by sympathetic nerve-induced vasoconstriction and thrombosis. The cells of the injured tissue release alarm signals, chemokines and growth factors, recruit immune cells from the blood circulation, stimulate the proliferation of resident cells in the tissue, and cause immune cells to accumulate in the wound site. The proliferation stage is characterized by the formation of granulation tissue, which is composed of newly formed blood vessels, immune cells and fibroblasts, and allows epidermal cells to migrate to this tissue during the re-epithelialization of the wound. In the process of scar tissue formation, fibroblasts in the dermis and epidermis deposit new extracellular matrix to strengthen the repaired tissue. The skin structure and composition of different species are different, and the speed of healing is also different.

At present, most local treatments for wound healing include functional dressings (e.g., interactive dressings and bioactive dressings), biological materials (e.g., xenogeneic acellular dermal matrix and tissue engineered skin), and negative pressure treatment techniques. Among the existing oral drugs, most of the auxiliary aspects of wound care, such as pain management, infection relief, and nutrition, are extremely lacking in oral drugs that directly promote wound healing. Because oral preparations are easy to use and accelerate healing time, increasing effective wound healing oral preparations will be very beneficial to the field of wound care.

Studies have reported that compared with the control group, the open wound area of rats' skin wounds injected with growth factors is reduced. In addition, microscopic observation showed that the vascular endothelial growth factor, fibroblast growth factor and insulin growth factor increased in the wound where the growth factor was injected, which promoted the growth of epithelial cells and angiogenesis, accelerated fibrous proliferation and the deposition of type I collagen, thereby Speed up wound healing.

In another study, the results of in vitro vascular remodeling evaluation on the aorta of WT and CypD KO mice showed that the onset of CypD KO mice was earlier and the wound closure speed was significantly faster, which was related to the increased distribution of blood vessels around the wound 14 days after ear removal.

WS635 (the compound of formula (I)) is a non-immunosuppressive derivative of CsA (cyclosporin A) double-substituted at the 3 and 4 positions that binded to CypD (Cyclophilin D) to inhibit the opening of mPTP. Therefore, WS635 warrants further investigation as a novel type of mPTP inhibitor.
Kong Wuyi et al., Journal of Cellular Physiology (2006), 210, 153-160 disclose a study about the role of cyclophilin C-associated protein (CyCAP) in wound healing. CN 108 348 468 A describes nanoparticles for the systemic or topical delivery of lipophilic diagnostic or therapeutic agents to a subject in need thereof. CN 110 678 187 A refers to senolytic compounds. CN 106 902 347 A teaches the application of a cyclophilin inhibitor. WO 2021/068188 A1 discloses uses of WS-635 in medicine. Yao Guiyang et al., Journal of Integration Technology (2015), 4, 28-44 describe nonimmunosuppressive cyclophilin inhibitors derived from the cyclosporine scaffolds.

### SUMMARY OF THE DISCLOSURE

In the research and development process, the inventors surprisingly found that WS635 could significantly promote angiogenesis in rats. The inventors further investigated that the WS635 could be used to promoting a healing process of wound. WS635 as the active ingredient of the healing wounds therapeutic agents has less toxic effects, which the NOAEL (No Observed Adverse Effect Level) dose was 80mg/kg/day for toxicity test of repeated oral administration for 9 days in rats. Moreover, WS635 has better stability, pharmacokinetics etc., and WS635 has already been shown to be more effective in healing wounds.

Specifically, in one aspect, the present invention relates to a compound of Formula I or a tautomer, a solvate or a pharmaceutically acceptable salt thereof for use in a method of wound in a subject in need thereof, the method comprising the step of administering to the subject a therapeutically effective amount of the compound of Formula I or a tautomer, a solvate, or a pharmaceutically acceptable salt thereof,

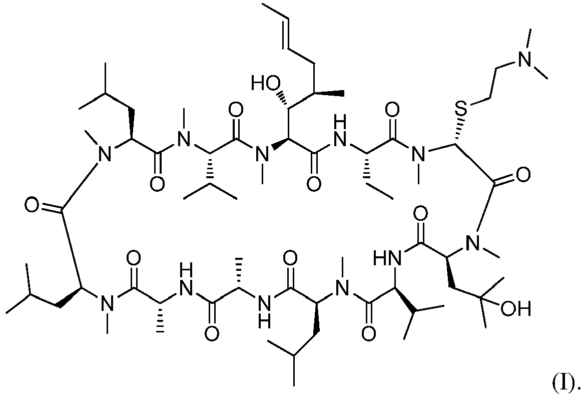

In the research and development process, the inventors surprisingly found that WS635 could promote angiogenesis in rats. According to the examples of present disclosure, the said compound of formula (I) can promote angiogenesis and promote a healing wound.

In one embodiment, the wound is a surgery-induced wound.

In one embodiment, the wound is a skin wound.

In one embodiment, the compound is administered to the subject after the wound formed.

In one embodiment, the compound is administered to the subject within 14 days after the wound formed.

In one embodiment, the compound is administered to the subject within 7 days after the wound formed.

In one embodiment, the compound is administered at a daily dose of less than about 1000mg.

In one embodiment, the compound is administered at a daily dose of between about 30 to about 1000mg.

In one embodiment, the compound is administered at a daily dose of between about 500 to about 700mg.

In one embodiment, the compound is administered 1 time per day.

In one embodiment, the compound is administered 1 time per day as a single dosage.

In one embodiment, the compound is administered orally.

In one embodiment, the compound is administered in a form of tablet, capsule or injection.

In one embodiment, the compound is administered in combination with one or more other agent used for inducing or accelerating a healing process of wound other than the compound of Formula I.

Any embodiment disclosed herein can be combined with other embodiments as long as they are not contradictory to one another, even though the embodiments are described under different aspects of the invention. In addition, any technical feature in one embodiment can be applied to the corresponding technical feature in other embodiments as long as they are not contradictory to one another, even though the embodiments are described under different aspects of the invention.

### BRIEF DESCRIPTION OF THE FIGURES

Fig. 1 shows that WS635 significantly promotes wound healing, and its effect is equivalent to rb-bFGF.
Fig. 2 shows that WS635 enhances wound healing rate in a time-dependent manner.

### DESCRIPTION OF THE DISCLOSURE

### DEFINITIONS AND GENERAL TERMINOLOGY

Reference will now be made in detail to certain embodiments of the invention, examples of which are illustrated in the accompanying structures and formulas. The references to the methods of treatment of this description are to be interpreted as references to compounds, pharmaceutical compositions and medicaments of the present disclosure for use in those methods.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all scientific and technical terms used herein have the same meaning as is commonly understood by one skilled in the art to which this invention belongs.

The grammatical articles "a", "an" and "the", as used herein, are intended to include "at least one" or "one or more" unless otherwise indicated herein or clearly contradicted by the context. Thus, the articles used herein refer to one or more than one (*i.e.* at least one) of the grammatical objects of the article. For example, "an embodiment" refers to one or more embodiments.

The term "comprise" is an open expression, it means comprising the contents disclosed herein, but don't exclude other contents.

The term "pharmaceutically acceptable" as used herein, refers to those compounds, materials, compositions, and/or dosage forms which are, within the scope of sound medical judgment, suitable for use in contact with the tissues of patients without excessive toxicity, irritation, allergic response, or other problem or complication commensurate with a reasonable benefit/risk ratio, and are effective for their intended use.

The term "prodrug" refers to a compound that is transformed in vivo into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic (C1-24) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound.

A "pharmaceutically acceptable salts" refers to organic or inorganic salts of a compound disclosed herein. Pharmaceutically acceptable salts are well known in the art. For example, S. M. Berge et al., describe pharmaceutically acceptable salts in detail in J. Pharmaceutical Sciences, 1977, 66: 1-19. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate, and the like. Salts derived from appropriate bases include alkali metal, alkaline earth metal, ammonium and N+(C1-4 alkyl)4 salts. This invention also envisions the quaternization of any basic nitrogen-containing groups of the compounds disclosed herein. Water or oil soluble or dispersible products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, C1-8 sulfonate or aryl sulfonate.

The term "solvate" refers to an association or complex of one or more solvent molecules and a compound disclosed herein. Examples of solvents that form solvates include, but are not limited to, water, isopropanol, ethanol, methanol, DMSO, ethyl acetate, acetic acid, ethanolamine and the mixture thereof. The term "hydrate" refers to the complex where the solvent molecule is water.

The term "hydrate" can be used when said solvent is water. In one embodiment, one water molecule is associated with one molecule of the compounds disclosed herein, such as a hydrate. In another embodiment, more than one water molecule may be associated with one molecule of the compounds disclosed herein, such as a dihydrate. In still another embodiment, less than one water molecule may be associated with one molecule of the compounds disclosed herein, such as a hemihydrate. Furthermore, all the solvates of the invention retain the biological effectiveness of the non-hydrate form of the compounds disclosed herein.

As used herein, the term "therapeutically effective amount" or "therapeutically effective dose" refers to the amount of compound disclosed herein that can elicit the biological or medical response (such as reducing or inhibiting the activity of an enzyme or protein, or improving symptoms, lessening disorders, slowing or delaying the development of diseases and the like).

### USE OF THE COMPOUNDS AND PHARMACEUTICAL COMPOSITIONS

Compounds WS635 or pharmaceutical compositions disclosed herein are efficient for promoting healing wound.

An "effective amount", "a therapeutically effective amount" or "effective dose" of the compound, or a stereoisomer, a tautomer, an N-oxide, a solvate, a metabolite, a pharmaceutically acceptable salt or a prodrug thereof or pharmaceutically acceptable composition disclosed herein is an amount that is effective promoting healing wound. The complex and pharmaceutically acceptable compositions are effectively administered in a fairly wide dose range. For example, the daily dose is from about 30 to about 1000mg per person, the compounds or pharmaceutically acceptable compositions can be administered in a single dose or in several divided doses a day. The compound and compositions, according to the method disclosed herein, may be administered using any amount and any route of administration which is effective for promoting healing wound. The exact amount required will vary from subject to subject, depending on the species, age, and general condition of the subject, the severity of the infection, the particular agent, its mode of administration, and the like.

In some embodiments, the compound described herein can be administered to a subject after the wound formed, for example within 14 days after the wound is formed. The compound described herein can be administered at a daily dose of less than about 1000mg, for example, at a daily dose of between about 30 to about 1000mg or at a daily dose of between about 500 to about 700mg.The compound can be administered 1 time per day or can be administered 1 time per day as a single dosage.

Besides being useful for human treatment, WS635 and the compositions thereof are also useful for veterinary treatment of animals such as companion animals, exotic animals and mammals of farm animals. In other embodiments, the animals disclosed herein include horses, dogs, and cats.

### Examples

### Materials and Methods

Eighteen 6-week-old male SD rats were randomly divided into 3 groups: model group, test article group and positive control group, with 6 rats in each group. Skin wound surgery was performed after anesthesia, and oral administration was started 2 h after skin wound formation. All rats were raised in a single cage. All rats were raised in a single cage. The surgical details were as follows: All animals were anesthetized with 10% chloral hydrate solution (3 mL/kg) by intraperitoneal injection, their backs were depatured with pet shaving knives, and a circular mark with a diameter of 2 cm was made. After the skin was disinfected with alcohol, the full layer of skin was removed with surgical scissors along the marking line, and the wound surface was pressed to stop bleeding. The model group was orally given 10%DMSO corn oil, the test group was orally given WS635(corn oil dissolved in 10%DMSO) at 52.8mg/kg, and the positive control group was given 262.5 IU/cm2(62.5 µL/cm²) recombinant bovine basic fibroblast growth factor (rb-bFGF) external solution, once a day for 7 days. Digital photos were taken of the wound of each rat under the same conditions. The wound was recorded with plastic transparent paper, and the wound area was scanned by image J software after the wound was recorded with plastic transparent paper. Wound healing rate was calculated according to wound area (%)=(area after administration - area before administration)/ area before administration 100%.

### Result

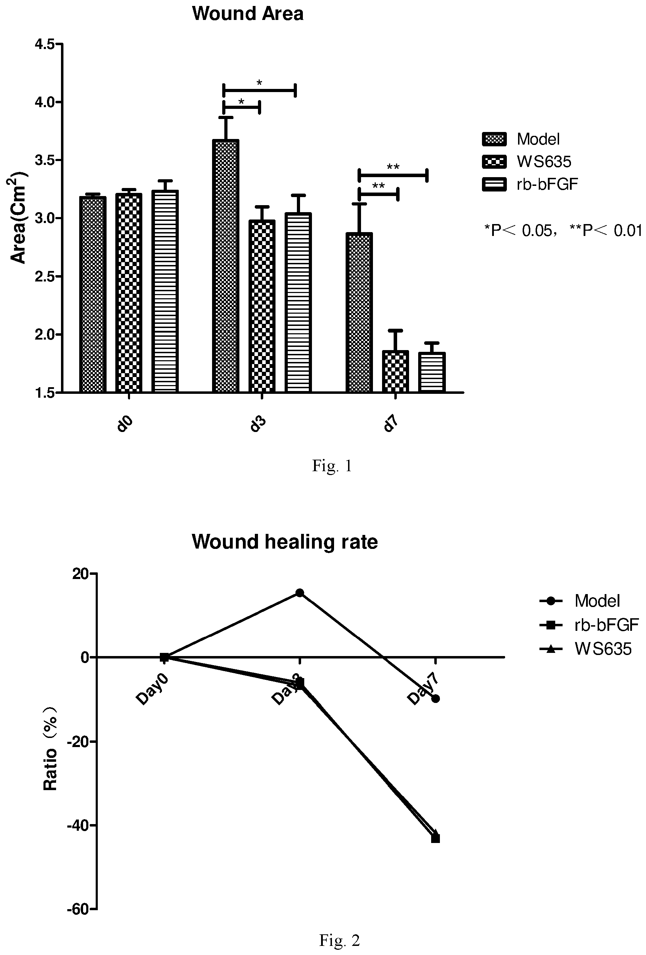

Compared with the model group, the wound area of WS635 and positive control (rb-bFGF) decreased significantly on Day 3 and Day 7 (P < 0.05, P < 0.01). WS635 and rb-bFGF had no significant difference (P > 0.05). Within the administration cycle, the wound healing rate of WS635 and rb-bFGF groups increased with the extension of administration time. The results showed that oral administration of WS635 can significantly promote wound healing in the wound healing model of SD rats. The results are shown in Figure 1 and Figure 2.

## Claims

1. A compound of Formula I or a tautomer, a solvate or a pharmaceutically acceptable salt thereof for use in a method of promoting a healing process of wound in a subject in need thereof, the method comprising the step of administering to the subject a therapeutically effective amount of the compound of Formula I or the tautomer, solvate or pharmaceutically acceptable salt thereof,

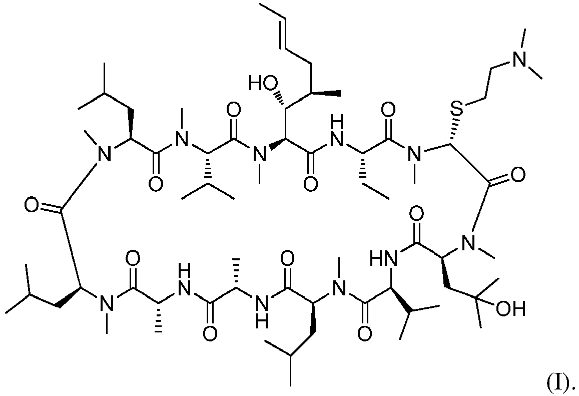

2. The compound for use according to claim 1, wherein the wound is a surgery-induced wound.

3. The compound for use according to claim 1, wherein the wound is a skin wound.

4. The compound for use according to claim 1, wherein the compound is administered to the subject after the wound formed.

5. The compound for use according to claim 1, wherein the compound is administered to the subject within 14 days after the wound formed.

6. The compound for use according to claim 1, wherein the compound is administered to the subject within 7 days after the wound formed.

7. The compound for use according to claim 1, wherein the compound is administered at a daily dose of less than about 1000mg.

8. The compound for use according to claim 1, wherein the compound is administered at a daily dose of between about 30 to about 1000mg.

9. The compound for use according to claim 1, wherein the compound is administered at a daily dose of between about 500 to about 700mg.

10. The compound for use according to claim 1, wherein the compound is administered 1 time per day.

11. The compound for use according to claim 1, wherein the compound is administered 1 time per day as a single dosage.

12. The compound for use according to claim 1, wherein the compound is administered orally .

13. The compound for use according to claim 1, wherein the compound is administered in a form of tablet, capsule or injection.

14. The compound for use according to claim 1, wherein the compound is administered in combination with one or more other agent used for inducing or accelerating a healing process of wound other than the compound of Formula I.

## Patentansprüche

1. Verbindung der Formel I oder Tautomer, Solvat oder pharmazeutisch akzeptables Salz davon zur Verwendung in einem Verfahren zur Förderung eines Heilungsprozesses einer Wunde bei einem Patienten, wobei das Verfahren den Schritt der Verabreichung einer therapeutisch wirksamen Menge der Verbindung der Formel I oder des Tautomers, Solvats oder pharmazeutisch akzeptables Salzes davon an den Patienten umfasst,

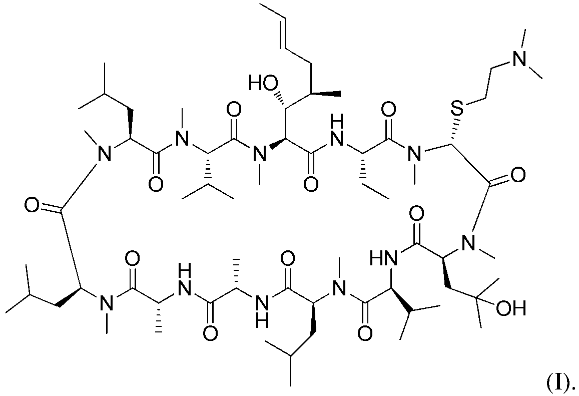

2. Verbindung zur Verwendung nach Anspruch 1, wobei die Wunde eine chirurgisch bedingte Wunde ist.

3. Verbindung zur Verwendung nach Anspruch 1, wobei die Wunde eine Hautwunde ist.

4. Verbindung zur Verwendung nach Anspruch 1, wobei die Verbindung dem Patienten nach der Wundbildung verabreicht wird.

5. Verbindung zur Verwendung nach Anspruch 1, wobei die Verbindung dem Patienten innerhalb von 14 Tagen nach der Wundbildung verabreicht wird.

6. Verbindung zur Verwendung nach Anspruch 1, wobei die Verbindung dem Patienten innerhalb von 7 Tagen nach der Wundbildung verabreicht wird.

7. Verbindung zur Verwendung nach Anspruch 1, wobei die Verbindung in einer Tagesdosis von weniger als etwa 1000 mg verabreicht wird.

8. Verbindung zur Verwendung nach Anspruch 1, wobei die Verbindung in einer Tagesdosis von etwa 30 bis etwa 1000 mg verabreicht wird.

9. Verbindung zur Verwendung nach Anspruch 1, wobei die Verbindung in einer Tagesdosis von etwa 500 bis etwa 700 mg verabreicht wird.

10. Verbindung zur Verwendung nach Anspruch 1, wobei die Verbindung 1 Mal pro Tag verabreicht wird.

11. Verbindung zur Verwendung nach Anspruch 1, wobei die Verbindung 1 Mal pro Tag als Einzeldosis verabreicht wird.

12. Verbindung zur Verwendung nach Anspruch 1, wobei die Verbindung oral verabreicht wird.

13. Verbindung zur Verwendung nach Anspruch 1, wobei die Verbindung in Form von Tabletten, Kapseln oder Injektionen verabreicht wird.

14. Verbindung zur Verwendung nach Anspruch 1, wobei die Verbindung in Kombination mit einem oder mehreren anderen Mitteln verabreicht wird, die zum Auslösen oder Beschleunigen eines Heilungsprozesses einer Wunde verwendet werden, die nicht die Verbindung der Formel I sind.

## Revendications

1. Un composé de Formule I ou un tautomère, un solvate, ou un sel pharmaceutiquement acceptable de celui-ci pour utilisation dans une méthode de promotion du processus de cicatrisation d'une plaie chez un sujet en ayant besoin, la méthode comprenant l'étape d'administration au sujet d'une quantité thérapeutiquement efficace du composé de Formule I ou du tautomère, solvate, ou sel pharmaceutiquement acceptable de celui-ci,

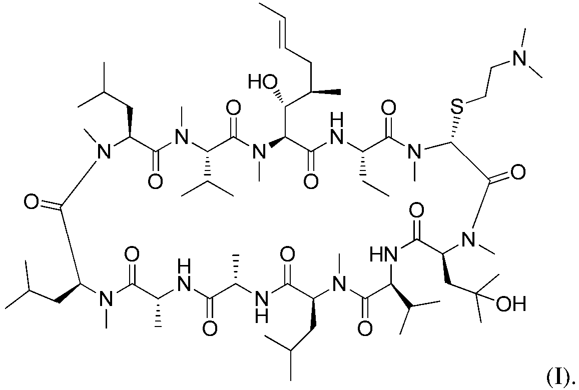

2. Le composé pour utilisation selon la revendication 1, où la plaie est une plaie induite par chirurgie.

3. Le composé pour utilisation selon la revendication 1, où la plaie est une plaie cutanée.

4. Le composé pour utilisation selon la revendication 1, où le composé est administré au sujet après la formation de la plaie.

5. Le composé pour utilisation selon la revendication 1, où le composé est administré au sujet dans les 14 jours suivant la formation de la plaie.

6. Le composé pour utilisation selon la revendication 1, où le composé est administré au sujet dans les 7 jours suivant la formation de la plaie.

7. Le composé pour utilisation selon la revendication 1, où le composé est administré à une dose quotidienne inférieure à environ 1 000 mg.

8. Le composé pour utilisation selon la revendication 1, où le composé est administré à une dose quotidienne comprise entre environ 30 et environ 1 000 mg.

9. Le composé pour utilisation selon la revendication 1, où le composé est administré à une dose quotidienne comprise entre environ 500 et environ 700 mg.

10. Le composé pour utilisation selon la revendication 1, où le composé est administré une fois par jour.

11. Le composé pour utilisation selon la revendication 1, où le composé est administré une fois par jour en une dose unique.

12. Le composé pour utilisation selon la revendication 1, où le composé est administré par voie orale.

13. Le composé pour utilisation selon la revendication 1, où le composé est administré sous forme de comprimé, de capsule ou d'injection.

14. Le composé pour utilisation selon la revendication 1, où le composé est administré en combinaison avec un ou plusieurs autres agents utilisés pour induire ou accélérer le processus de cicatrisation d'une plaie autre que le composé de Formule I.
